# FASCICULE DE BREVET EUROPEEN

(11) **EP 0 914 442 B1**
(45) Date de publication et mention de la délivrance du brevet: **22.09.2004**
(21) Numéro de dépôt: 97934579.0
(22) Date de dépôt: 16.07.1997
(51) Int. Cl.: C12N 15/38, C12N 15/45, C12N 15/40, C12N 15/34, C12N 15/50, C12N 15/48, C12N 15/44, A61K 39/295

(54) **FORMULE DE VACCIN POLYNUCLEOTIDIQUE AVIAIRE**
HÜHNER POLYNUKLEOTIDE IMPFSTOFF-FORMULIERUNG
AVIAN POLYNUCLEOTIDE VACCINE FORMULA

(30) Priorité: 19.07.1996 FR 9609339
(43) Date de publication de la demande: 12.05.1999
(73) Titulaire: MERIAL, 69002 Lyon (FR)
(72) Inventeur: AUDONNET, Jean-Christophe, F-69006 Lyon (FR); BOUCHARDON, Annabelle, F-69007 Lyon (FR); RIVIERE, Michel, F-69130 Ecully (FR)
(74) Mandataire: Nargolwalla, Cyra
(86) Numéro de dépôt international: PCT/FR1997/001326
(87) Numéro de publication internationale: WO 1998/003659

(56) Documents cités:
- WO-A-95/20660
- WO-A-96/12808
- WO-A-96/21034

## Description

La présente invention a trait à un vaccin permettant la vaccination des espèces aviaires, en particulier poulet contre la maladie de Newcastle et éventuellement contre un ou plusieurs autres pathogènes aviaires.

On a déjà proposé par le passé des associations de vaccins contre un certain nombre de virus responsables de pathologies chez le poulet.

Les associations développées jusqu'à présent étaient réalisées à partir de vaccins inactivés ou de vaccins vivants. Leur mise en oeuvre pose des problèmes de compatibilité entre valences et de stabilité. Il faut en effet assurer à la fois la compatibilité entre les différentes valences de vaccin, que ce soit au plan des différents antigènes utilisés ou au plan des formulations elles-mêmes. Il se pose également le problème de la conservation de tels vaccins combinés et de leur innocuité notamment en présence d'adjuvant. Ces vaccins sont en général assez coûteux.

WO-A-96/21034 est représentatif d'un autre type de vaccins, utilisant un virus recombiné vecteur comportant et exprimant *in vivo* le gène codant pour un immunogène du pathogène contre lequel la vaccination est dirigée. Dans ce document, le virus vecteur est un virus de la maladie de Marek, e.g. HVT, et on y décrit l'insertion de gènes de pathogènes aviaires, par exemple les gènes F et HN du virus de la maladie de Newcastle.

Les demandes de brevet WO-A-90 11092, WO-A-93 19183, WO-A-94 21797 et WO-A-95 20660 ont fait usage de la technique récemment développée des vaccins polynueléotidiques. On sait que ces vaccins utilisent un plasmide apte à exprimer dans les cellules de l'hôte l'antigène inséré dans le plasmide. Toutes les voies d'administration ont été proposées (incrapériconéale, intraveineuse, intramusculaire, transcutanée, intradermique, mucosale, etc.). Différents moyens de vaccination peuvent également être utilisés, tels que ADN déposé à la surface de particules d'or et projeté de façon à pénétrer dans la peau de l'animal (Tang et al,. Nature 356, 152-154, 1992) et les injecteurs par jet liquide permettant de transfecter à la fois dans la peau, le muscle, les tissus graisseux et les tissus mammaires (Furth et al., Analytical Biochemistry, 205, 365-368, 1992).

Les vaccins polynucléotidiques peuvent utiliser aussi bien des ADN nus que des ADN formulés par exemple au sein de liposomes ou de lipides cationiques.

WO-A-98/12808 concerne ce type de vaccin et décrit un protocole de vaccination contre la maladie de Newcastle. Les auteurs comparent un plasmide circulaire exprimant le gène F et des plasmides linéaires exprimant ce même gène. Les résultats montrent l'absence de protection avec le plasmide circulaire. L'enseignement de cet exemple, ainsi que l'enseignement global de ce document, est qu'un plasmide circulaire contenant un gène du virus de la maladie de Newcastle ne constitue pas une solution adaptée à la vaccination de l'espèce aviaire contre cette maladie. Il est au contraire préconisé d'utiliser des plasmides linéarisés.

Contrairement à cet enseignement, le demandeur a trouvé qu'un plasmide circulaire exprimant un gène du virus de la maladie de Newcastle permet de produire un vaccin polynucléotide efficace.

L'invention se propose donc de fournir un vaccin permettant d'assurer une vaccination contre la maladie de Newcastle.

Un autre objectif de l'invention est de fournir un vaccin associant d'autres valences tout en présentant tous les critères requis de compatibilité et de stabilité des valences entre elles.

Un autre objectif de l'invention est de fournir un vaccin permettant d'associer différentes valences dans un même véhicule.

Un autre objectif de l'invention est de fournir un tel vaccin qui soit de mise en oeuvra aisée et peu coûteuse.

Un autre objectif, encore de l'invention est de fournir une méthode de vaccination des gallinacés qui permette d'obtenir une protection, y compris une protection multivalente, avec un niveau élevé d'efficacité et de longue durée, ainsi qu'une bonne innocuité et une absence de résidus

La présente invention a pour objet un vaccin aviaire comprenant un plasmide circulaire contenant le gène HN du virus de la maladie de Newcastle, et un véhicule approprié.

La présente invention peut y associer au moins un autre plasmide intégrant, de manière à l'exprimer in vivo dans les cellules hôtes, un gène d'une autre valence de pathogène aviaire, cette valence étant choisie parmi le groupe consistant en virus de la maladie de Marek (MDV), virus de la maladie de Gumboro (IBDV), virus de la bronchite infectieuse (IBV), virus de l'anémie infectieuse (CAV), virus de la laryngotrachéite infectieuse (ILTV), virus de l'encéphalomyélite (AEV ou encore virus de la leucose aviaire ALV), virus de la pneumovirose, et virus de la peste aviaire, les plasmides comprenant, pour chaque valence, un ou plusieurs des gènes choisis parmi le groupe consistant en gB et gD pour le virus de la maladie de Marek, HN et F pour le virus de la maladie de Newcastle, VP2 pour le virus de la maladie de Gumboro, S, M et N pour le virus de la bronchite infectieuse, C + NS1 pour le virus de l'anémie infectieuse, gB et gD pour le virus de la laryngotrachéite infectieuse, env et gag/pro pour le virus de l'encéphalomyélite, F et G pour le virus de la pneumovirose et HA, N et NP pour le virus de la peste aviaire.

Par valence, dans la présente invention, on entend au moins un antigène assurant une protection contre le virus du pathogène considéré, la valence pouvant contenir, à titre de sous-valence, un ou plusieurs gènes naturels ou modifiés d'une ou plusieurs souches du pathogène considéré.

Par gène d'agent pathogène, on entend non seulement le gène complet, mais aussi les séquences nueléocidiques différentes, y compris fragments, conservant la capacité à induire une réponse protectrice. La notion de gène recouvre les séquences nucléotidiques équivalentes à celles décrites précisément dans les exemples, c'est-à-dire les séquences différentes mais codant pour la même protéine. Elle recouvre aussi les séquences nucléotidiques d'autres souches du pathogène considéré, assurant une protection croisée ou une protection spécifique de souche ou de groupe de souche. Elle recouvre encore les séquences nucléotidiques qui ont été modifiées pour faciliter l'expression in vivo par l'animal hôte mais codant pour la même protéine.

De préférence, le vaccin selon l'invention comprend deux autres valences choisies parmi Marek, Gumboro et Anémie infectieuse. On peut aussi y rajouter de préférence la valence bronchite infectieuse.

Sur cette base de 3, 4 ou 5 valences, on pourra ajouter une ou plusieurs des valences poste aviaire, laryngotrachéite, pneumovirose et encéphalomyélite.

En ce qui concerne la valence Marek, on peut mettre en oeuvre les deux gènes codant pour gB et gD, dans des plasmides différents ou dans un seul et même plasmide. On préfère toutefois n'utiliser que le gène gB.

Pour la valence Newcastle, on utilise de préférence les deux gènes HN et F intégrés dans deux plasmides différents ou dans un seul et même plasmide.

Pour la valence bronchite infectieuse, on préfère utiliser le gène S. Eventuellement, mais de façon moins préférée, on peut associer S et M dans un seul plasmide ou dans des plasmides différents.

Pour la valence anémie infectieuse, on préfère associer les deux gènes C et NS1 dans le même plasmide.

Pour la valence laryngotrachéite infectieuse, on préfère utiliser le gène gB seul. Eventuellement, mais de façon moins préférée, on peut associer les deux gènes gB et gD dans des plasmides différents ou dans un seul et même plasmide.

Pour la valence pneumovirose, on préfère utiliser les deux gènes F et G, dans un seul plasmide ou dans des plasmides différents.

Pour la valence peste aviaire, on préfère utiliser le gène HA. Eventuellement, mais de façon moins préférée, on peut utiliser des associations HA et NP ou HA et N dans des plasmides différents ou dans un seul et même plasmide. De préférence, on associe dans le même vaccin les séquences HA de plus d'une souche de virus influenza, en particulier des différentes souches rencontrées sur le terrain. En revanche, NP assure une protection croisée et l'on pourra donc se contenter de la séquence d'une seule souche du virus.

Pour la valence encéphalomyélite, on préfère utiliser env.

Le vaccin selon l'invention pourra se présenter sous un volume de dose compris entre 0,1 et 1 ml et en particulier entre 0,3 et 0,5 ml.

La dose sera généralement comprise entre 10 ng et 1 mg, de préférence entre 100 ng et 500 µg et préférentiellement entre 0,1 µg et 50 µg par type de plasmide.

On utilisera de préférence des plasmides nus, simplement placés dans le véhicule de vaccination qui sera en général de l'eau physiologique ou analogue. on peut bien entendu utiliser toutes les formes de vaccin polynucléotidique décrites dans l'art antérieur et notamment formulés dans des liposomes.

Chaque plasmide comprend un promoteur apte à assurer l'expression du gène inséré sous sa dépendance dans les cellules hôtes. Il s'agira en général d'un promoteur eucaryote fort et en particulier d'un promoteur précoce du cytomégalovirus CMV-IE, d'origine humaine ou murine, ou encore éventuellement d'une autre origine telle que rat, cochon, cobaye.

De manière plus générale, le promoteur pourra être soit d'origine virale, soit d'origine cellulaire. Comme promoteur viral autre que CMV-IE, on peut citer le promoteur précoce ou tardif du virus SV 40 ou le promoteur LTR du virus du Sarcome de Rous. Il peut aussi s'agir d'un promoteur du virus dont provient le gène, par exemple le promoteur propre au gène.

Comme promoteur cellulaire, on peut citer le promoteur d'un gène du cytosquelette, tel que par exemple le promoteur de la desmine (Bolmont et al., Journal of Submicroscopic Cytology and Pathology, 1990, 22, 117-122 ; et ZHENLIN et al., Gene, 1989, 78, 243-254), ou encore le promoteur de l'actine.

Lorsque plusieurs gènes sont présents dans le même plasmide, ceux-ci peuvent être présentés dans la même unité de transcription ou dans deux unités différentes.

La combinaison des différentes valences du vaccin selon l'invention peut être effectuée, de préférence, par mélange de plasmides polynucléotidiques exprimant le ou les antigènes de chaque valence, mais on peut également prévoir de faire exprimer des antigènes de plusieurs valences par un même plasmide.

Les vaccins monovalents peuvent aussi être utilisés (i) pour la préparation d'un vaccin polyvalent tel que décrit plus haut, (ii) à titre individuel contre la pathologie propre, (iii) associés à un vaccin d'un autre type (entier vivant ou inactivé, recombinant, sous-unité) contre une autre pathologie, ou (iv) comme rappel d'un vaccin comme décrit ci-après.

La présente invention a en effet encore pour objet l'utilisation d'un ou de plusieurs plasmides selon l'invention pour la fabrication d'un vaccin aviaire destiné à vacciner des animaux primo-vaccinés au moyen d'un premier vaccin classique (monovalent ou multi-valent), du type de ceux de l'art antérieur, choisi notamment dans le groupe consistant en vaccin entier vivant, vaccin entier inactivé, vaccin de sous-unité, vaccin recombinant, ce premier vaccin présentant (c'est-à-dire contenant ou pouvant exprimer) le ou les antigène(s) codé(s) par le ou les plasmides ou antigène(s) assurant une protection croisée.

De manière remarquable, le vaccin polynucléocidique a un effet de rappel puissant se traduisant par une amplification de la réponse immunitaire et l'instauration d'une immunité de longue durée.

De manière générale, les vaccins de primo-vaccination pourront être choisis parmi les vaccins commerciaux disponibles auprès des différents producteurs de vaccins vétérinaires.

L'invention a aussi pour objet un kit de vaccination regroupant un vaccin selon l'invention et un vaccin de primo-vaccination tel que décrit ci-dessus. Elle a aussi trait à un vaccin selon l'invention accompagné d'une notice indiquant l'usage de ce vaccin comme rappel d'une primo-vaccination telle que décrite ci-avant.

On peut encore préciser une méthode de vaccination aviaire, comprenant l'administration d'un vaccin efficace tel que décrit plus haut. Cette méthode de vaccination comprend l'administration d'une ou de plusieurs doses de vaccin, ces doses pouvant être administrées successivement dans un court laps de temps et/ou successivement à des moments éloignés l'un de l'autre.

Les vaccins selon l'invention pourront être administrés, dans le cadre de cette méthode de vaccination, par les différentes voies d'administration proposées dans l'art antérieur dans le cadre de la vaccination polynucléocidique et au moyen des techniques d'administration connues.

On visera en particulier les voies intramusculaire, in ovo, intra-oculaire, la nébulisation et l'eau de boisson.

L'efficacité de la présentation des antigènes au système immunitaire varie en fonction des tissus. En particulier, les muqueuses de l'arbre respiratoire servent de barrière à l'entrée des pathogènes et sont associées à des tissus lymphoides qui supportent une immunité locale. En outre, l'administration d'un vaccin par contact avec les muqueuses, en particulier muqueuse buccale, pharyngée et région bronchique présente un intérêt certain pour la vaccination de masse.

En conséquence, les voies d'administration mucosales font partie d'un mode d'administration préféré pour l'invention, utilisant notamment la nébulisation ou spray ou l'eau de boisson. Les vaccins selon l'invention pourront être appliqués dans ce cadre.

L'invention prévoit encore une primo-vaccination telle que décrite ci-dessus et un rappel avec un vaccin selon l'invention.

Dans une forme de mise en oeuvre préférée du procédé selon l'invention, on administre dans un premier temps, à l'animal, une dose efficace du vaccin de type classique, notamment inactivé, vivant, atténué ou recombinant, ou encore un vaccin de sous-unité de façon à assurer une primo-vaccination, et, après un délai de préférence de 2 à 6 semaines, on assure l'administration du vaccin polyvalent ou monovalent selon l'invention.

L'invention concerne aussi la méthode de préparation des formules de vaccin, à savoir la préparation des valences et leurs mélanges, telle qu'elle ressort de cette description.

L'invention va être maintenant décrite plus en détails à l'aide de modes de réalisation de l'invention pris en référence aux dessins annexés.

### Liste des figures

- Figure N° 1 :: Plasmide pVR1012
- Figure N° 2 :: Plasmide pAB045
- Figure N° 3 :: Plasmide pAB080
- Figure N° 4 :: Séquence du gène NDV HN souche Texas GB
- Figure N° 5 :: Plasmide pAB046
- Figure N° 6 :: Séquence du gène NDV F souche Texas GB
- Figure N° 7 :: Plasmide pAB047
- Figure N° 8 :: Séquence du gène IBDV VP2 souche Faragher
- Figure N° 9 :: Plasmide pAB048
- Figure N° 10 :: Séquence du gène IBV S souche Massachusetts 41
- Figure N° 11 :: Plasmide pAB049
- Figure N° 12 :: Séquence du gène IBV M souche Massachusetts 41
- Figure N° 13 :: Plasmide pAB050
- Figure N° 14 :: Séquence du gène IBV N souche Massachusetts 41
- Figure N° 15 :: Plasmide pAB051
- Figure N° 16 :: Plasmide pAB054
- Figure N° 17 :: Plasmide pAB055
- Figure N° 18 :: Plasmide pAB076
- Figure N° 19 :: Plasmide pAB089
- Figure N° 20 :: Plasmide pAB086
- Figure N° 21 :: Plasmide pAB081
- Figure N° 22 :: Plasmide pAB082
- Figure N° 23 :: Plasmide pAB077
- Figure N° 24 :: Plasmide pAB078
- Figure N° 25 :: Plasmide pAB088
- Figure N° 26 :: Plasmide pAB079

### Liste des séquences SEQ ID N°

SEQ ID N° 1 : Oligonucléotide AB062
SEQ ID N° 2 : Oligonucléotide AB063
SEQ ID N° 3 : Oligonucléotide AB148
SEQ ID N° 4 : Oligonucléotide AB149
SEQ ID N° 5 : Oligonucléotide AB072
SEQ ID N° 6 : Oligonucléotide AB073
SEQ ID N° 7 : Séquence du gène NDV HN souche Texas GB
SEQ ID N° 8 : Oligonucléotide AB091
SEQ ID N° 9 : Oligonucléotide AB092
SEQ ID N° 10 : Séquence du gène NDV F souche Texas GB
SEQ ID N° 11 : Oligonucléotide AB093
SEQ ID N° 12 : Oligonucléotide AB094
SEQ ID N° 13 : Séquence du "gène" IBDV VP2 souche Faragher
SEQ ID N° 14 : Oligonucléotide AB095
SEQ ID N° 15 : Oligonucléotide AB096
SEQ ID N° 16 : Séquence du gène IBV S souche Massachusetts 41
SEQ ID N° 17 : Oligonucléotide AB097
SEQ ID N° 18 : Oligonucléotide AB098
SEQ ID N° 19 : Séquence du gène IBV M souche Massachusetts 41
SEQ ID N° 20 : Oligonucléotide AB099
SEQ ID N° 21 : Oligonucléotide AB100
SEQ ID N° 22 : Séquence du gène IBV N souche Massachusetts 41
SEQ ID N° 23 : Oligonucléotide CD064
SEQ ID N° 24 : Oligonucléotide CD065
SEQ ID N° 25 : Oligonucléotide CD066
SEQ ID N° 26 : Oligonucléotide AB105
SEQ ID N° 27 : Oligonucléotide AB140
SEQ ID N° 28 : Oligonucléotide AB141
SEQ ID N° 29 : Oligonucléotide AB164
SEQ ID N° 30 : Oligonucléotide AB165
SEQ ID N° 31 : Oligonucléotide AB160
SEQ ID N° 32 : Oligonucléotide AB161
SEQ ID N° 33 : Oligonucléotide AB1 50
SEQ ID N° 34 : Oligonucléotide AB151
SEQ ID N° 35 : Oligonucléotide AB152
SEQ ID N° 36 : Oligonucléotide AB153
SEQ ID N° 37 : Oligonucléotide AB142
SEQ ID N° 38 : Oligonucléotide AB143
SEQ ID N° 39 : Oligonucléotide AB144
SEQ ID N° 40 : Oligonucléotide AB145
SEQ ID N° 41 : Oligonucléotide AB156
SEQ ID N° 42 : Oligonucléotide AB158
SEQ ID N° 43 : Oligonucléotide AB146
SEQ ID N° 44 : Oligonucléotide AB147

### EXEMPLES

### Exemple 1 : Culture des virus

Les virus sont cultivés sur le système cellulaire approprié jusqu'à obtention d'un effet cytopathique. Les systèmes cellulaires à utiliser pour chaque virus sont bien connus de l'homme du métier. Brièvement, des cellules sensibles au virus utilisé, cultivées en milieu minimum essentiel de Eagle (milieu "MEM) ou un autre milieu approprié, sont inoculées avec la souche virale étudiée en utilisant une multiplicité d'infection de 1. Les cellules infectées sont alors incubées à 37°C pendant le temps nécessaire à l'apparition d'un effet cytopathique complet (en moyenne 36 heures).

### Exemple 2 : Extraction des ADNs génomiques viraux

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM). Cette suspension virale concentrée est traitée par la protéinase K (100 µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile. Il peut alors être digéré par des enzymes de restriction.

### Exemple 3 : Isolement des ARNs génomiques viraux

Les virus à ARN ont été purifiés selon les techniques bien connues de l'homme du métier. L'ARN viral génomique de chaque virus a été ensuite isolé en utilisant la technique d'extraction "thiocyanate de guanidium/phénol-chloroforme" décrite par P. Chomczynski et N. Sacchi (Anal. Biochem. 1987. **162.** 156-159).

### Exemple 4 : Techniques de biologie moléculaire

Toutes les constructions de plasmides ont été réalisées en utilisant les techniques standards de biologie moléculaire décrites par J. Sambrook *et al*. (*Molecular Cloning: A Laboratory Manual*. 2^{nd} Edition. Cold Spring Harbor Laboratory. Cold Spring Harbor. New York. 1989). Tous les fragments de restriction utilisés pour la présente invention ont été isolés en utilisant le kit "Geneclean" (BIO101 Inc. La Jolla, CA).

### Exemple 5 : Technique de RT-PCR

Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (voir exemples spécifiques). La réaction de transcription inverse (RT) et l'amplification en chaîne par polymérase (PCR) ont été effectuées selon les techniques standards (Sambrook J. *et al*. 1989). Chaque réaction de RT-PCR a été faite avec un couple d'amplimers spécifiques et en prenant comme matrice l'ARN génomique viral extrait. L'ADN complémentaire amplifié a été extrait au phénol/chloroforme/alcool isoamylique (25:24:1) avant d'être digéré par les enzymes de restriction.

### Exemple 6 : plasmide pVR1012

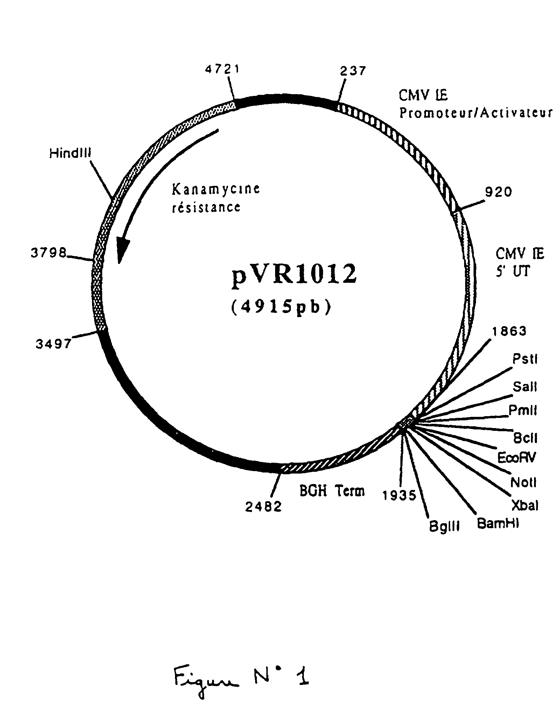

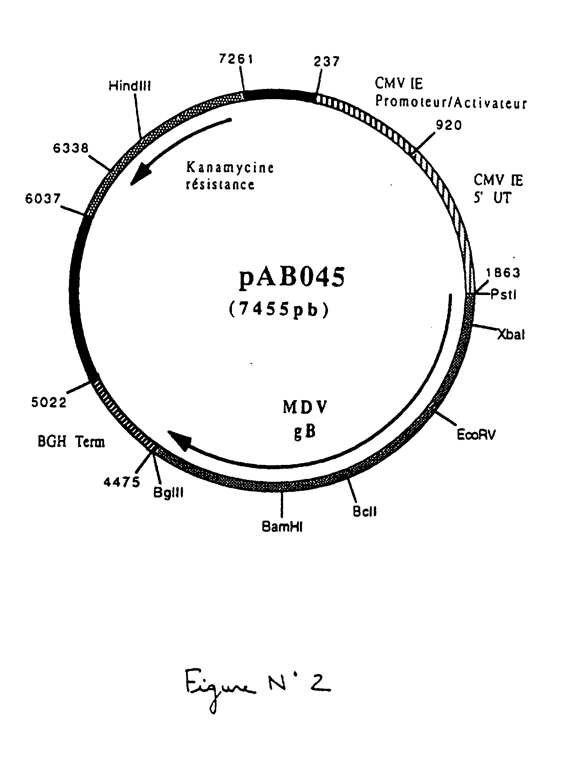

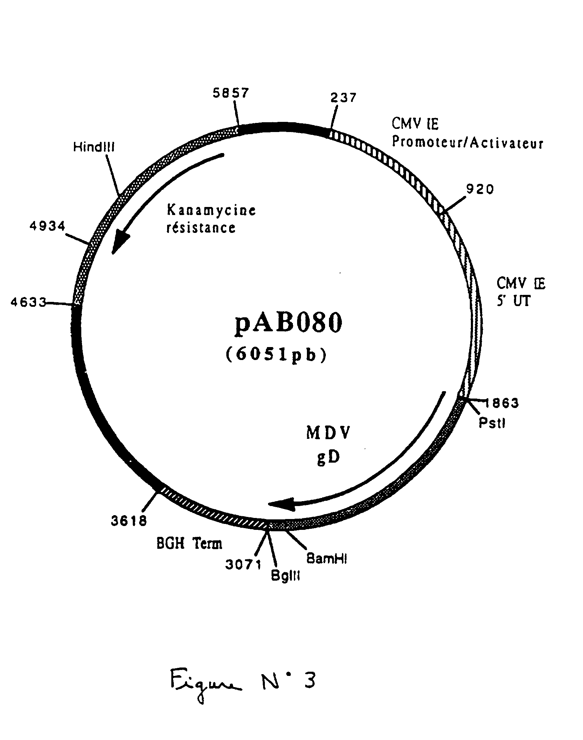

Le plasmide pVR1012 (Figure N° 1) a été obtenu auprès de Vical Inc. San Diego, CA, USA. Sa construction a été décrite dans J. Hartikka *et al*. (Human Gene Therapy. 1996. **7**. 1205-1217).

### Exemple 7 : Construction du plasmide pAB045 (gène MDV gB)

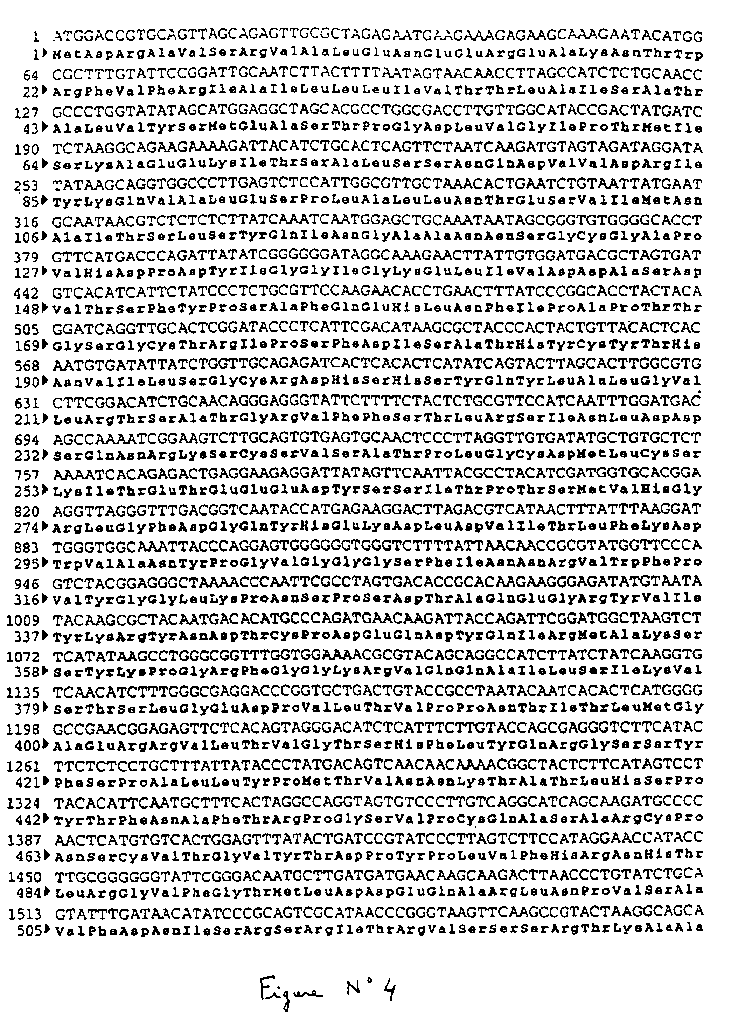

Une réaction de PCR a été réalisée avec l'ADN génomique du virus de la maladie de Marek (MDV) (Souche RB1B) (L. Ross *et al*. J. Gen. Virol. 1989. 70. 1789-1804), préparé selon la technique de l'exemple 2, et avec les oligonucléotides suivants: 
AB062 (37 mer) (SEQ ID N° 1)
5'AAAACTGCAGACTATGCACTATTTTAGGCGGAATTGC 3'
 
AB063 (35 mer) (SEQ ID N° 2)
5'GGAAGATCTTTACACAGCATCATCTTCTGAGTCTG 3'
 pour isoler le gène codant pour la glycoprotéine gB du virus MDV sous la forme d'un fragment PstI-BglII. Après purification, le produit de PCR de 2613 pb a été digéré par *Pst*I et *Bgl*II pour isoler un fragment PstI-BglII de 2602 pb. Ce fragment a été ligaturé avec le vecteur pVR1012 (exemple 6), préalablement digéré avec *Pst*I et *Bgl*II, pour donner le plasmide pA8045 (7455 pb) (Figure N° 2).

### Exemple 8 : Construction du plasmide pAB080 (gène MDV gD)

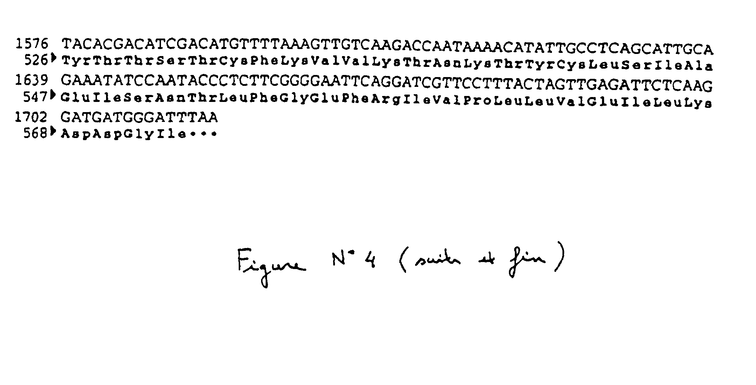

Une réaction de PCR a été réalisée avec l'ADN génomique du virus de la maladie de Marek (MDV) (Souche RB1B) (L. Ross *et al*. J. Gen. Virol. 1991. 72. 949-954), préparé selon la technique de l'exemple 2, et avec les oligonucléotides suivants: 
AB148 (29 mer) (SEQ ID N° 3)
5'AAACTGCAGATGAAAGTATTTTTTTTTAG 3'
 
AB149 (32 mer) (SEQ ID N° 4)
5'GGAAGATCTTTATAGGCGGGAATATGCCCGTC 3'
 pour isoler le gène codant pour la glycoprotéine gD du virus MDV sous la forme d'un fragment PstI-BglII. Après purification, le produit de PCR de 1215 pb a été digéré par *Pst*I et *Bgl*II pour isoler un fragment PstI-BglII de 1199 pb. Ce fragment a été ligaturé avec le vecteur pVR1012 (exemple 6), préalablement digéré avec *Pst*I et *Bgl*II, pour donner le plasmide pAB080 (6051 pb) (Figure N° 3).

### Exemple 9 : Construction du plasmide pAB046 (gène NDV HN)

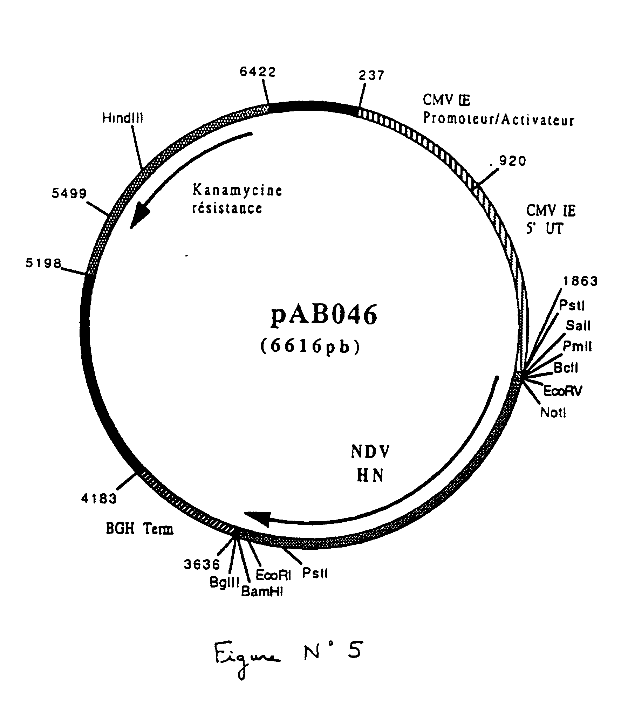

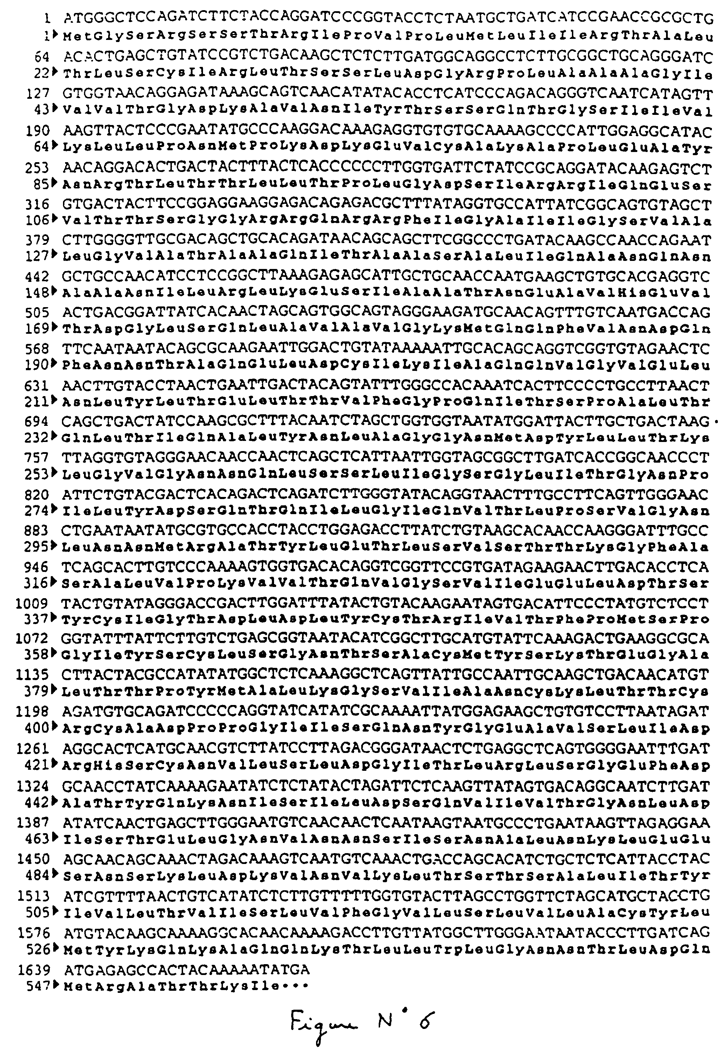

Une réaction de RT-PCR selon la technique de l'exemple 5 a été réalisée avec l'ARN génomique du virus de la maladie de Newcastle (NDV) (Souche Texas GB), préparé selon la technique de l'exemple 3, et avec les oligonucléotides suivants: 
AB072 (39 mer) (SEQ ID N° 5)
5'ATAAGAATGCGGCCGCCATGGACCGTGCAGTTAGCAGAG 3'
 
AB073 (34 mer) (SEQ ID N° 6)
5'CGCGGATCCTTAAATCCCATCATCCTTGAGAATC 3'
 pour isoler le gène codant pour la glycoprotéine HN du virus NDV souche Texas GB (Figure N° 4 et SEQ ID N° 7) sous la forme d'un fragment NotI-BamHI. Après purification, le produit de RT-PCR de 1741 pb a été digéré par *Not*I et *Bam*HI pour isoler un fragment NotI-BamHI de 1723 pb. Ce fragment a été ligaturé avec le vecteur pVR1012 (exemple 6), préalablement digéré avec *Not*I et *Bam*HI, pour donner le plasmide pAB046 (6616 pb) (Figure N° 5).

### Exemple 10 : Construction du plasmide pAB047 (gène NDV F)

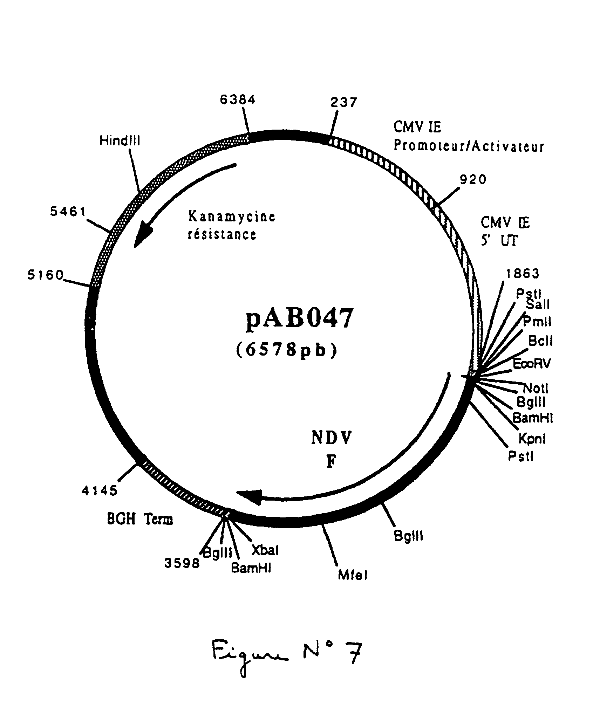

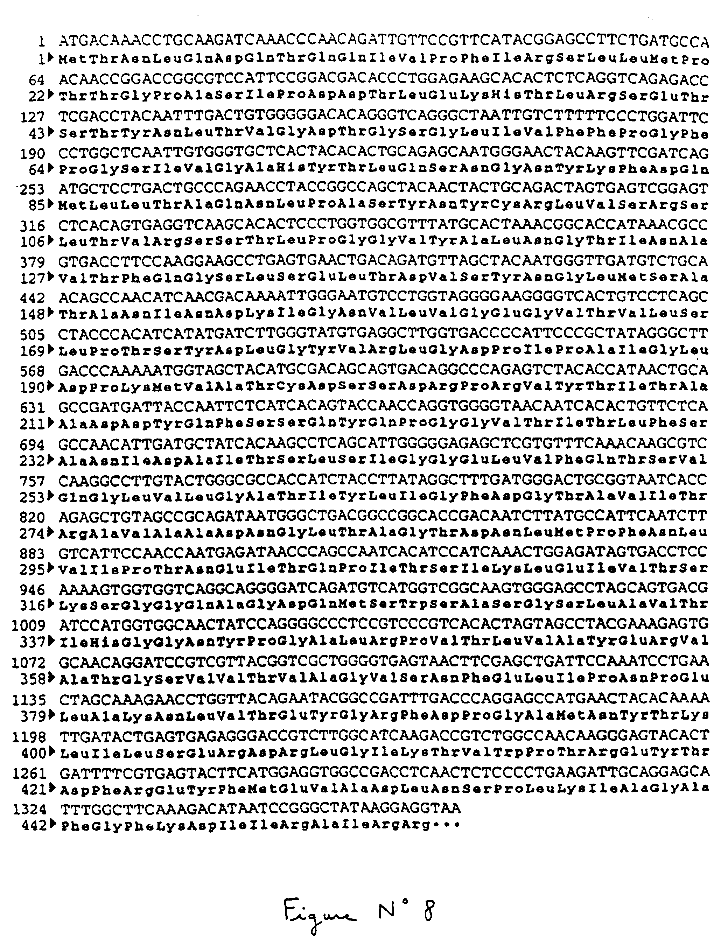

Une réaction de RT-PCR selon la technique de l'exemple 5 a été réalisée avec l'ARN génomique du virus de la maladie de Newcastle (NDV) (Souche Texas GB), préparé selon la technique de l'exemple 3, et avec les oligonucléotides suivants: 
AB091 (37 mer) (SEQ ID N° 8)
5'AGAATGCGGCCGCGATGGGCTCCAGATCTTCTACCAG 3'
 
AB092 (34 mer) (SEQ ID N° 9)
5'TGCTCTAGATCATATTTTTGTAGTGGCTCTCATC 3'
 pour isoler le gène codant pour la glycoprotéine F du virus NDV souche Texas GB (Figure N° 6 et SEQ ID N° 10) sous la forme d'un fragment NotI-XbaI. Après purification le produit de RT-PCR de 1684 pb a été digéré par *Not*I et *Xba*l pour isoler un fragment NotI-XbaI de 1669 pb. Ce fragment a été ligaturé avec le vecteur pVR1012 (exemple 6), préalablement digéré avec *Not*I et *Xba*I, pour donner le plasmide pAB047 (6578 pb), (Figure N° 7).

### Exemple 11 : Construction du plasmide pAB048 (gène IBDV VP2)

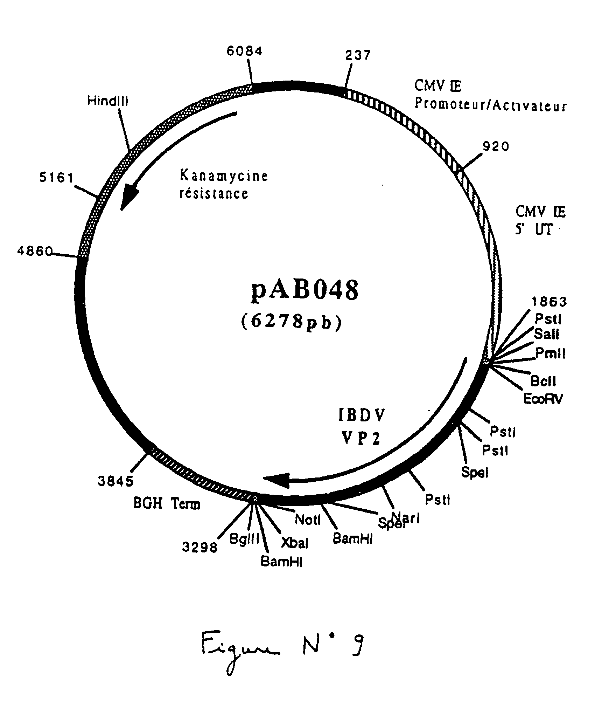

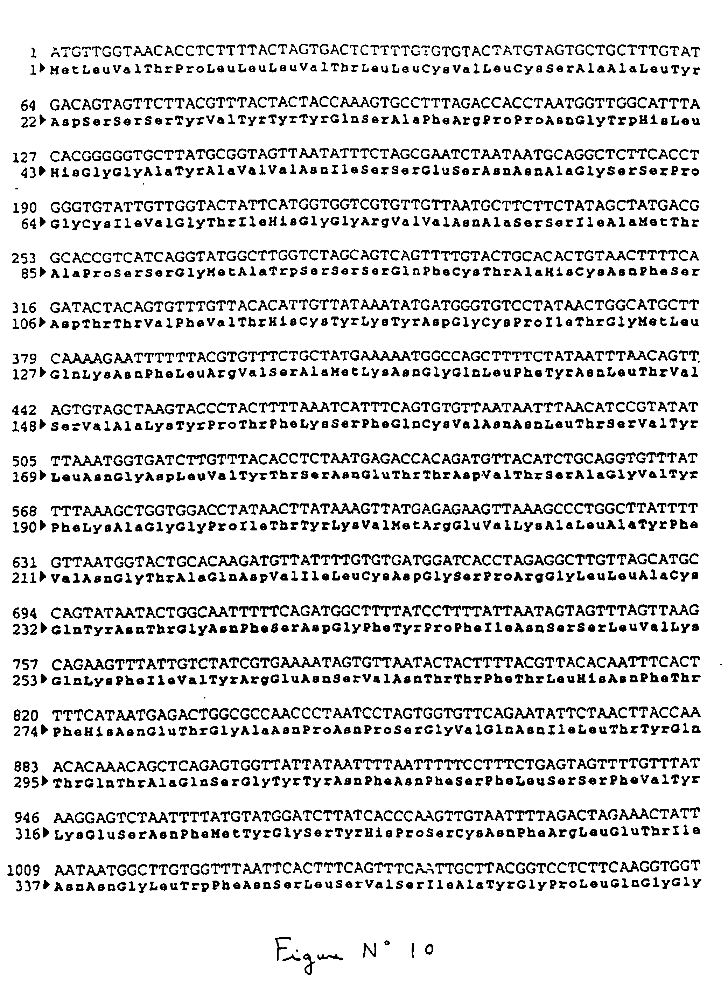

Une réaction de RT-PCR selon la technique de l'exemple 5 a été réalisée avec l'ARN génomique du virus de la maladie de Gumboro (IBDV) (Souche Faragher), préparé selon a technique de l'exemple 3, et avec les oligonucléotides suivants: 
AB093 (33 mer) (SEQ ID N° 11)
5'TCAGATATCGATGACAAACCTGCAAGATCAAAC 3'
 
AB094 (38 mer) (SEQ ID N° 12)
5'AGAATGCGGCCGCTTACCTCCTTATAGCCCGGATTATG 3'
 pour isoler la séquence codant pour la protéine VP2 du virus IBDV souche faragher (Figure N° 8 et SEQ ID N° 13) sous la forme d'un fragment EcoRV-NotI. Après purification, le produit de RT-PCR de 1384 pb a été digéré par *Eco*RV et *Not*I pour isoler un fragment EcoRV-NotI de 1367 pb. Ce fragment a été ligaturé avec le vecteur pVR1012 (exemple 6), préalablement digéré avec *Eco*RV et *Not*I, pour donner le plasmide pAB048 (6278 pb) (Figure N° 9).

### Exemple 12 : Construction du plasmide pAB049 (gène IBV S1)

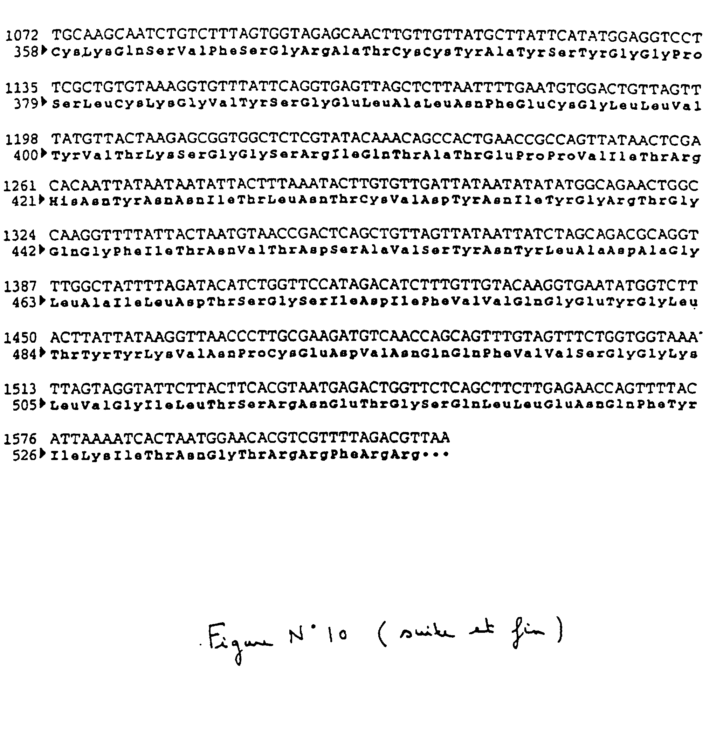

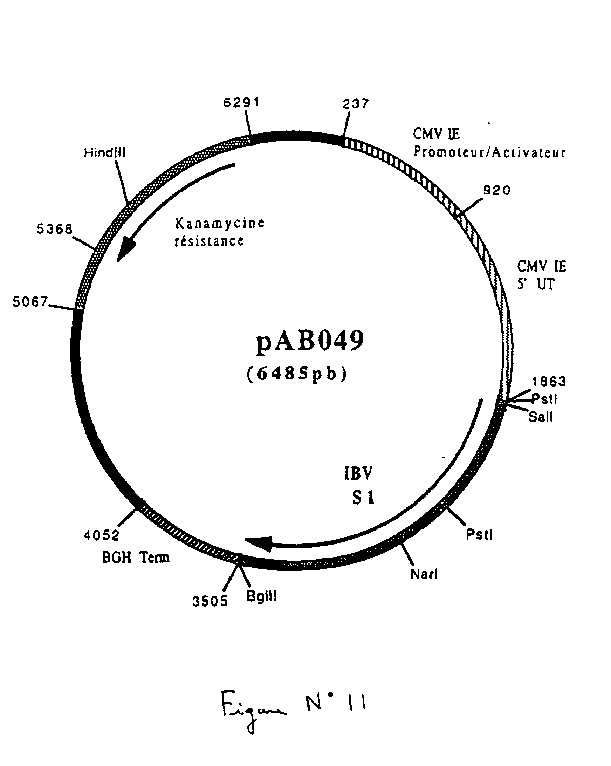

Une réaction de RT-PCR selon la technique de l'exemple 5 a été réalisée avec l'ARN génomique du virus de la bronchite infectieuse du poulet (IBV) (Souche Massachusetts 41), préparé selon a technique de l'exemple 3, et avec les oligonucléotides suivants: 
AB095 (32 mer) (SEQ ID N° 14)
5'ACGCGTCGACATGTTGGTAACACCTCTTTTAC 3'
 
AB096 (35 mer) (SEQ ID N° 15)
5'GGAAGATCTTCATTAACGTCTAAAACGACGTGTTC 3'
 pour isoler la séquence codant pour la sous-unité S1 de la glycoprotéine S du virus IBV souche Massachusetts 41 (Figure N° 10 et SEQ ID N° 16) sous la forme d'un fragment SalI-BglII. Après purification, le produit de RT-PCR de 1635 pb a été digéré par *Sal*I et *Bgl*II pour isoler un fragment SalI-BglII de 1622 pb. Ce fragment a été ligaturé avec le vecteur pVR1012 (exemple 6), préalablement digéré avec *Sal*I et *Bgl*II, pour donner le plasmide pAB049 (6485 pb) (Figure N° 11).

### Exemple 13 : Construction du plasmide pAB050 (gène IBV M)

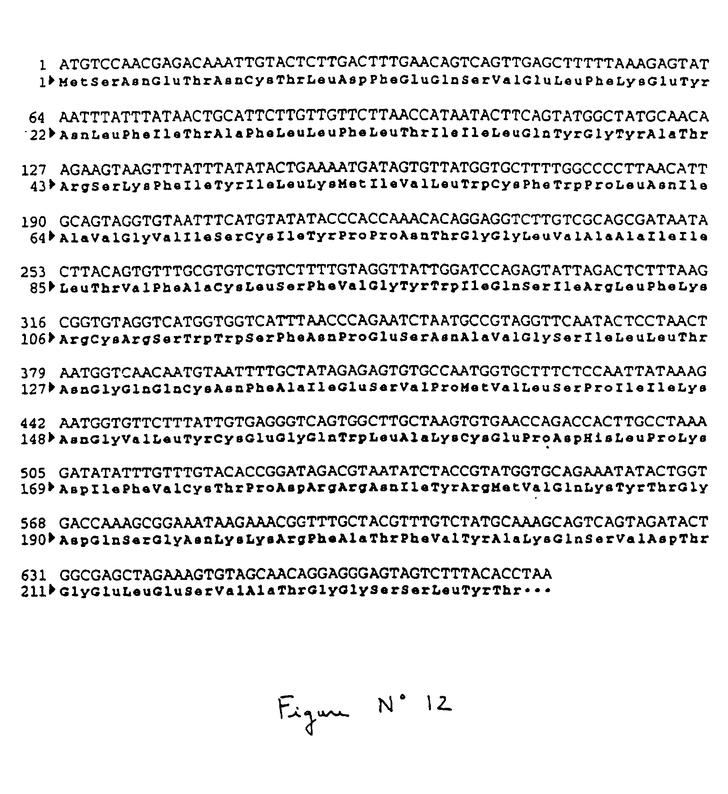

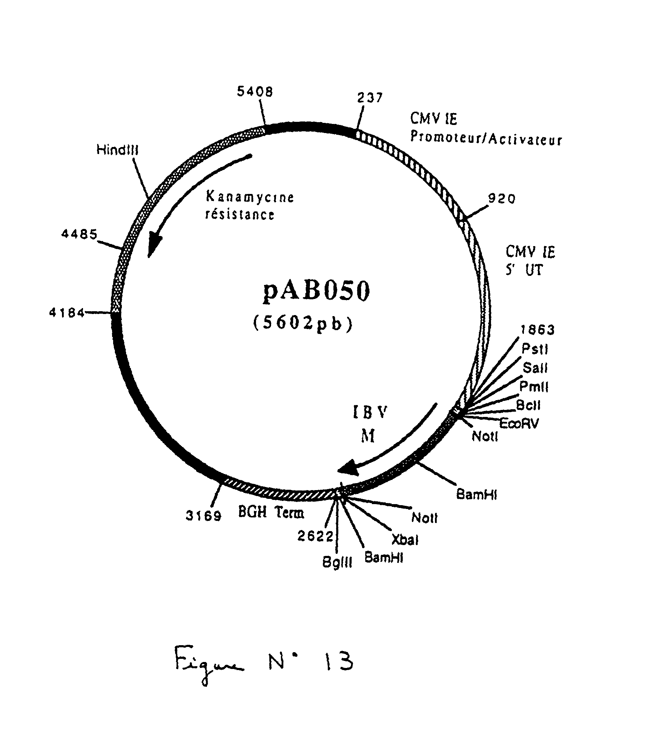

Une réaction de RT-PCR selon la technique de l'exemple 5 a été réalisée avec l'ARN génomique du virus de la bronchite infectieuse du poulet (IBV) (Souche Massachusetts 41), préparé selon la technique de l'exemple 3, et avec les oligonucléotides suivants: 
AB097 (37 mer) (SEQ ID N° 17)
5'ATAAGAATGCGGCCGCATGTCCAACGAGACAAATTGTAC 3'
 
AB098 (38 mer) (SEQ ID N° 18)
5'ATAAGAATGCGGCCGCTTTAGGTGTAAAGACTACTCCC 3'
 pour isoler le gène codant pour la glycoprotéine M du virus IBV souche Massachusetts 41 (Figure N° 12 et SEQ ID N° 19) sous la forme d'un fragment NotI-NotI. Après purification, le produit de RT-PCR de 710 pb a été digéré par *Not*I pour isoler un fragment NotI-NotI de 686 pb. Ce fragment a été ligaturé avec le vecteur pVR1012 (exemple 6), préalablement digéré avec *Not*I, pour donner le plasmide pAB050 (5602 pb) qui contient le gène IBV M dans la bonne orientation par rapport au promoteur (Figure N° 13).

### Exemple 14 : Construction du plasmide pAB051 (gène IBV N)

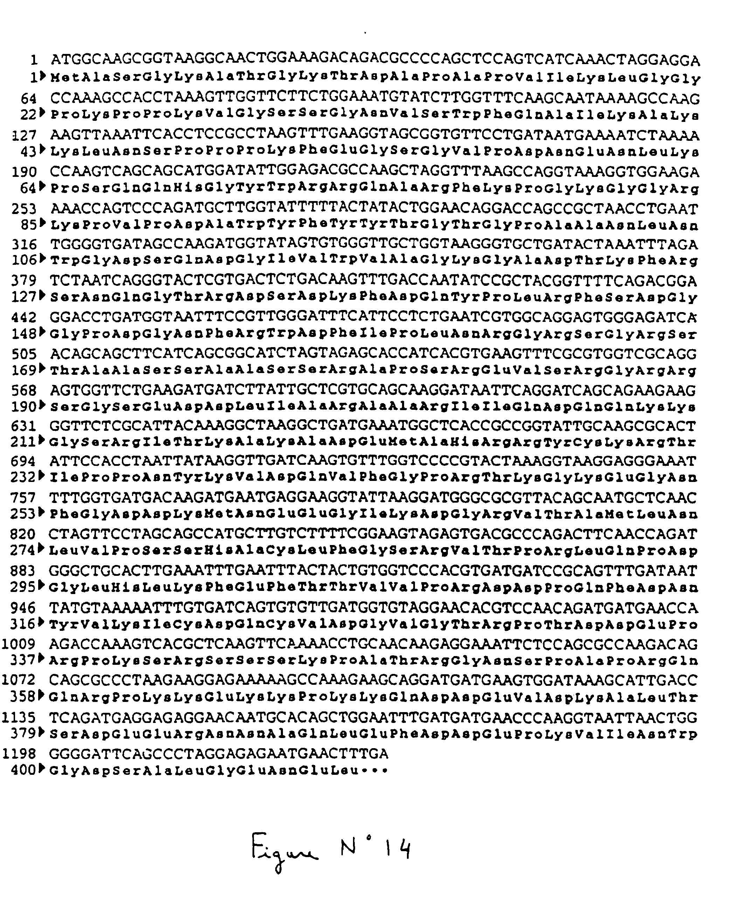

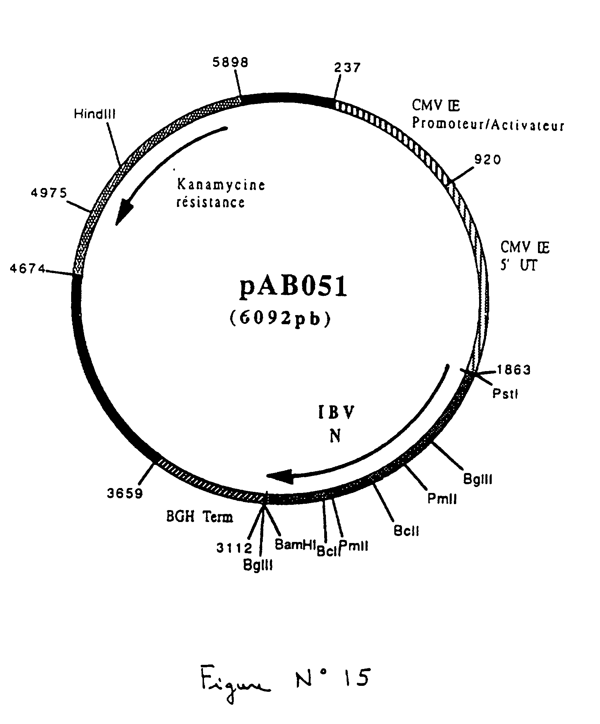

Une réaction de RT-PCR selon la technique de l'exemple 5 a été réalisée avec l'ARN génomique du virus de la bronchite infectieuse du poulet (IBV) (Souche Massachusetts 41), préparé selon la technique de l'exemple 3, et avec les oligonucléotides suivants: 
AB099 (34 mer) (SEQ ID N° 20)
5'AAAACTGCAGTCATGGCAAGCGGTAAGGCAACTG 3'
 
AB100 (33 mer) (SEQ ID N° 21)
5'CGCGGATCCTCAAAGTTCATTCTCTCCTAGGGC 3'
 pour isoler le gène codant pour la protéine N du virus IBV souche Massachusetts 41 (Figure N° 14 et SEQ ID N° 22) sous la forme d'un fragment PstI-BamHI. Après purification, le produit de RT-PCR de 1250 pb a été digéré par *Pst*I et *Bam*HI pour isoler un fragment PstI-BamHI de 1233 pb. Ce fragment a été ligaturé avec le vecteur pVR 1012 (exemple 6), préalablement digéré avec *Pst*I et *Bam*HI, pour donner le plasmide pAB051 (6092 pb) (Figure N° 15).

### Exemple 15 : Construction du plasmide pAB054 (gène CAV VP1)

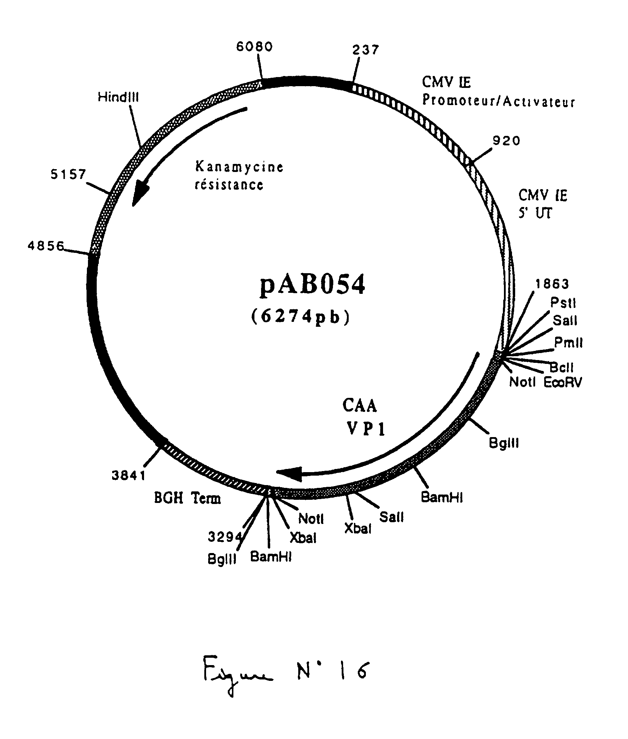

Une réaction de PCR a été réalisée avec l'ADN génomique du virus de l'anémie du poulet (CAV) (Souche Cuxhaven-1) (B. Meehan *et al*. Arch. Virol. 1992. **124.** 301-319), préparé selon la technique de l'exemple 2, et avec les oligonucléotides suivants: 
CD064 (39 mer) (SEQ ID N° 23)
5'TTCTTGCGGCCGCCATGGCAAGACGAGCTCGCAGACCGA 3'
 
CD065 (38 mer) (SEQ ID N° 24)
5'TTCTTGCGGCCGCTCAGGGCTGCGTCCCCCAGTACATG 3'
 pour isoler le gène codant pour la protéine de la capside VP1 du CAV sous la forme d'un fragment NotI-NotI. Après purification, le produit de PCR de 1377 pb a été digéré par *Not*l pour isoler un fragment NotI-NotI de 1359 pb. Ce fragment a été ligaturé avec le vecteur pVR1012 (exemple 6), préalablement digéré avec *Not*I, pour donner le plasmide pAB054 (6274 pb) qui contient le gène CAV VP1 dans la bonne orientation par rapport au promoteur (Figure N° 16).

### Exemple 17 : Construction du plasmide pAB055 (gène CAV VP2)

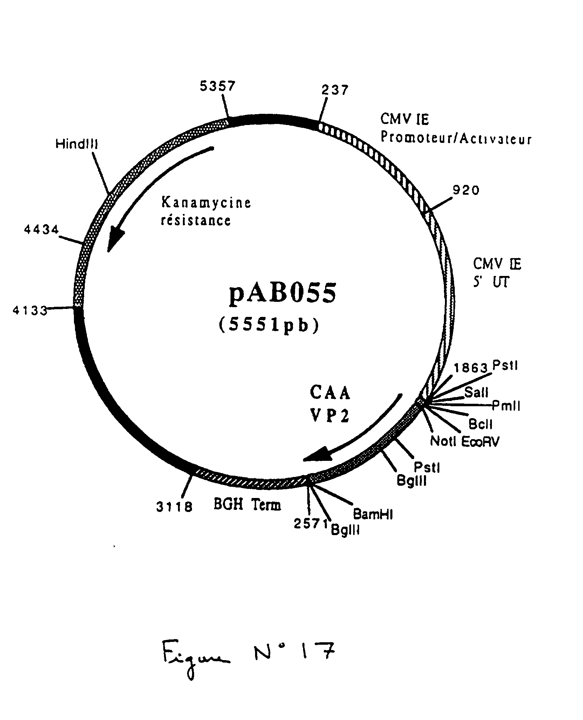

Une réaction de PCR a été réalisée avec l'ADN génomique du virus de l'anémie du poulet (CAV) (Souche Cuxhaven-1) (B. Meehan *et al*. Arch. Virol. 1992. **124.** 301-319), préparé selon la technique de l'exemple 2, et avec les oligonucléotides suivants: 
CD066 (39 mer) (SEQ ID N° 25)
5'TTCTTGCGGCCGCCATGCACGGGAACGGCGGACAACCGG 3'
 
AB105 (32 mer) (SEQ ID N° 26)
5'CGCGGATCCTCACACTATACGTACCGGGGCGG 3'
 pour isoler le gène codant pour la protéine VP2 du virus CAV sous la forme d'un fragment NotI-BamHI. Après purification, le produit de PCR de 674 pb a été digéré par *Not*I et *Bam*HI pour isoler un fragment NotI-BamHI de 659 pb. Ce fragment a été ligaturé avec le vecteur pVR1012 (exemple 6), préalablement digéré avec *Not*I et *Bam*HI, pour donner le plasmide pAB055 (5551 pb) (Figure N° 17).

### Exemple 18 : Construction du plasmide pAB076 (gène ILTV gB)

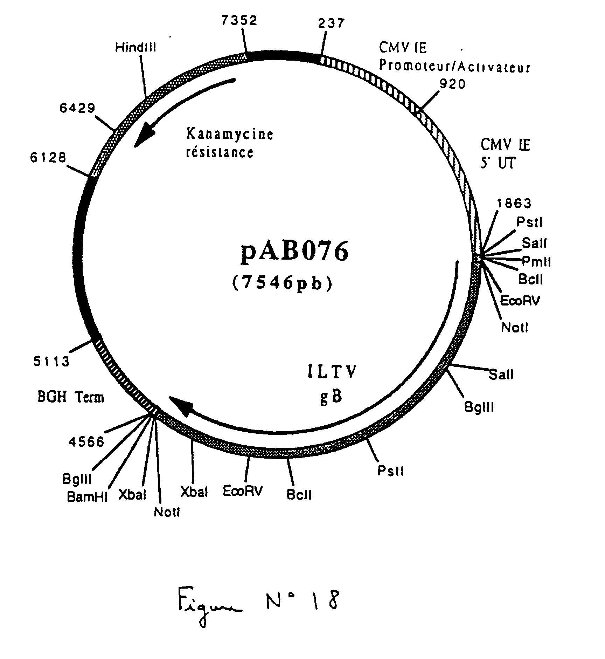

Une réaction de PCR a été réalisée avec l'ADN génomique du virus de la laryngotrachéite infectieuse du poulet (ILTV) (Souche SA-2) (K. Kongsuwan *et al*. Virology. 1991. 184. 404-410), préparé selon la technique de l'exemple 2, et avec les oligonucléotides suivants: 
AB140 (38 mer) (SEQ ID N° 27)
5'TTCTTGCGGCCGCCATGGCTAGCTTGAAAATGCTGATC 3'
 
AB141 (36 mer) (SEQ ID N° 28)
5'TTCTTGCGGCCGCTTATTCGTCTTCGCTTTCTTCTG 3'
 pour isoler le gène codant pour la glycoprotéine g8 du virus ILTV sous la forme d'un fragment NotI-NotI. Après purification, le produit de PCR de 2649 pb a été digéré par *Not*I pour isoler un fragment NotI-NotI de 2631 pb. Ce fragment a été ligaturé avec le vecteur pVR1012 (exemple 6), préalablement digéré avec *Not*I, pour donner le plasmide pAB076 (7546 pb) qui contient le gène ILTV gB dans la bonne orientation par rapport au promoteur (Figure N° 18).

### Exemple 20 : Construction du plasmide pAB089 (gène ILTV gD)

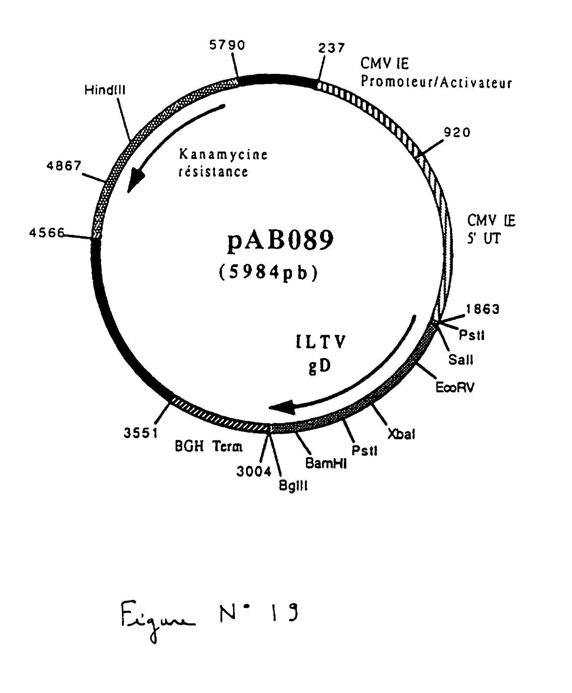

Une réaction de PCR a été réalisée avec l'ADN génomique du virus de la laryngotrachéite infectieuse du poulet (ILTV) (Souche SA-2) (M. Johnson *et al*. 1994. N° d'accès séquence sur Genbank = L31965), préparé selon la technique de l'exemple 2, et avec les oligonucléotides suivants: 
AB164 (33 mer) (SEQ ID N° 29)
5'CCGGTCGACATGGACCGCCATTTATTTTTGAGG 3'
 
AB165 (33 mer) (SEQ ID N° 30)
5'GGAAGATCTTTACGATGCTCCAAACCAGTAGCC 3'
 pour isoler le gène codant pour la glycoprotéine gD du virus ILTV sous la forme d'un fragment SalI-BglII. Après purification, le produit de PCR de 1134 pb a été digéré par *Sal*I et *Bgl*II pour isoler un fragment SalI-BglII de 1122 pb. Ce fragment a été ligaturé avec le vecteur pVR1012 (exemple 6), préalablement digéré avec *Sal*I et *Bgl*II, pour donner le plasmide pAB089 (5984 pb) (Figure N° 19).

### Exemple 21 : Construction du plasmide pAB086 (gène AEV env)

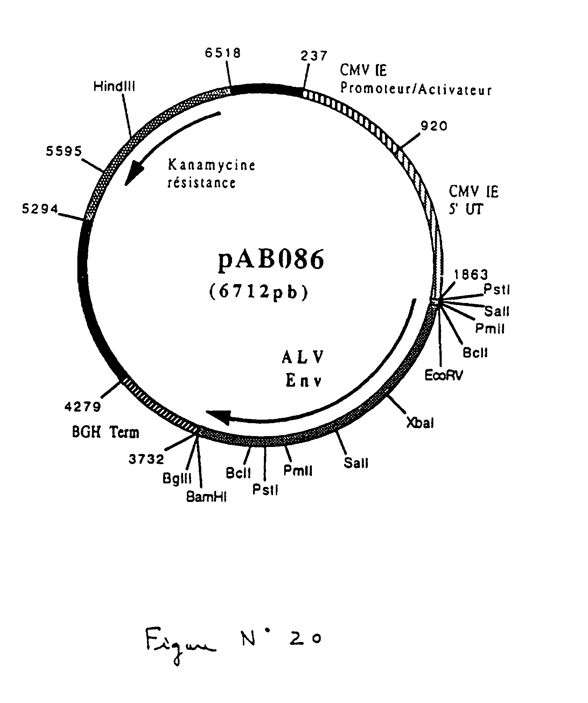

Une réaction de RT-PCR selon la technique de l'exemple 5 a été réalisée avec l'ARN génomique du virus de l'encéphalomyélite aviaire (AEV) (Type C) (E. Bieth *et al*. Nucleic Acids Res. 1992. 20. 367), préparé selon la technique de l'exemple 3, et avec les oligonucléotides suivants: 
AB160 (54 mer) (SEQ ID N° 31)
5'TTTGATATCATGGAAGCCGTCATTAAGGCATTTCTGACTGGATACCCTGG
GAAG 3'
 
AB161 (31 mer) (SEQ ID N° 32)
5'TTTGGATCCTTATACTATTCTGCTTTCAGGC 3'
 pour isoler la séquence codant pour la glycoprotéine Env du virus AEV sous la forme d'un fragment EcoRV-BamHI. Après purification, le produit de RT-PCR de 1836 pb a été digéré par *Eco*RV et *Bam*HI pour isoler un fragment EcoRV-BamHI de 1825 pb. Ce fragment a été ligaturé avec le vecteur pVR1012 (exemple 6), préalablement digéré avec EcoRV et BamHI, pour donner le plasmide pAB086 (6712 pb) (Figure N° 20).

### Exemple 22 : Construction du plasmide pAB081 (gène AEV gag/pro)

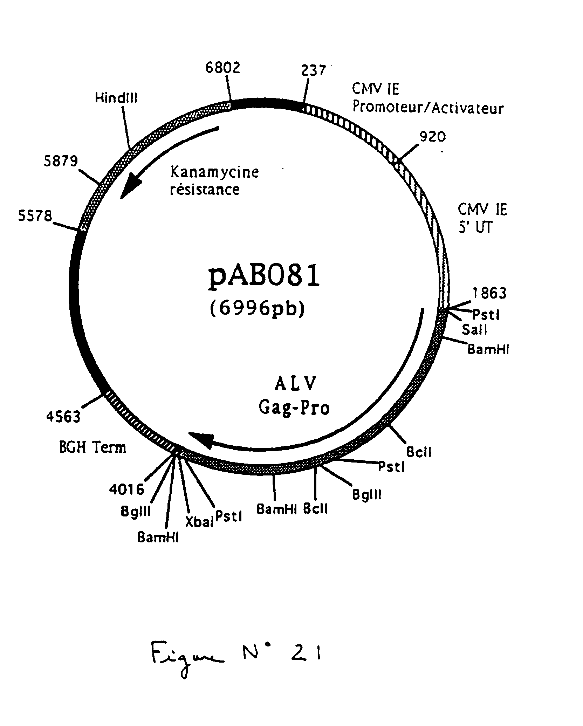

Une réaction de RT-PCR selon la technique de l'exemple 5 a été réalisée avec l'ARN génomique du virus de l'encéphalomyélite aviaire (AEV) (Type C) (E. Bieth *et al*. Nucleic Acids Res. 1992. 20. 367), préparé selon la technique de l'exemple 3, et avec les oligonucléotides suivants: 
AB150 (31 mer) (SEQ ID N° 33)
5'ACGCGTCGACATGGAAGCCGTCATTAAGGTG 3'
 
AB151 (32 mer) (SEQ ID N° 34)
5'TGCTCTAGACTATAAATTTGTCAAGCGGAGCC 3'
 pour isoler la séquence codant pour les protéines Gag et Pro du virus AEV sous la forme d'un fragment SalI-XbaI. Après purification, le produit de RT-PCR de 2125 pb a été digéré par *Sal*I et *Xba*l pour isoler un fragment SalI-XbaI de 2111 pb. Ce fragment a été ligaturé avec le vecteur pVR1012 (exemple 6), préalablement digéré avec *Sal*I et *Xba*l, pour donner le plasmide pAB081 (6996 pb) (Figure N° 21).

### Exemple 23 : Construction du plasmide pAB082 (gène Pneumovirus G)

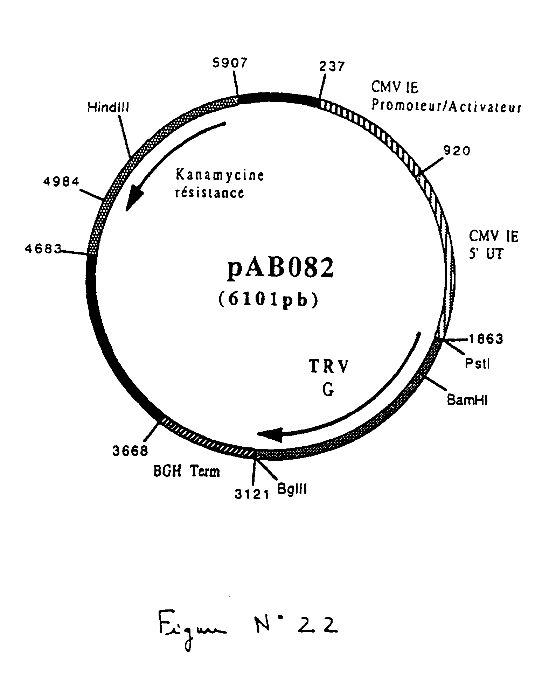

Une réaction de RT-PCR selon la technique de l'exemple 5 a été réalisée avec l'ARN génomique du virus de la rhinotrachéite de la dinde (TRV) (Souche 2119) (K. Juhasz *et al*. J. Gen. Virol. 1994, **75**. 2873-2880), préparé selon la technique de l'exemple 3, et avec les oligonucléotides suivants: 
AB152 (32 mer) (SEQ ID N° 35)
5'AAACTGCAGAGATGGGGTCAGAGCTCTACATC 3'
 
AB153 (31 mer) (SEQ ID N° 36)
5'CGAAGATCTTTATTGACTAGTACAGCACCAC 3'
 pour isoler le gène codant pour la glycoprotéine G du virus TRV sous la forme d'un fragment PstI-BglII. Après purification, le produit de RT-PCR de 1265 pb a été digéré par *Pst*I et *Bgl*II pour isoler un fragment PstI-BglII de 1249 pb. Ce fragment a été ligaturé avec le vecteur pVR1012 (exemple 6), préalablement digéré avec *Pst*I et *Bgl*II, pour donner le plasmide pAB082 (6101 pb) (Figure N° 22).

### Exemple 24 : Construction du plasmide pAB077 (gène Peste aviaire HA souche H2N2)

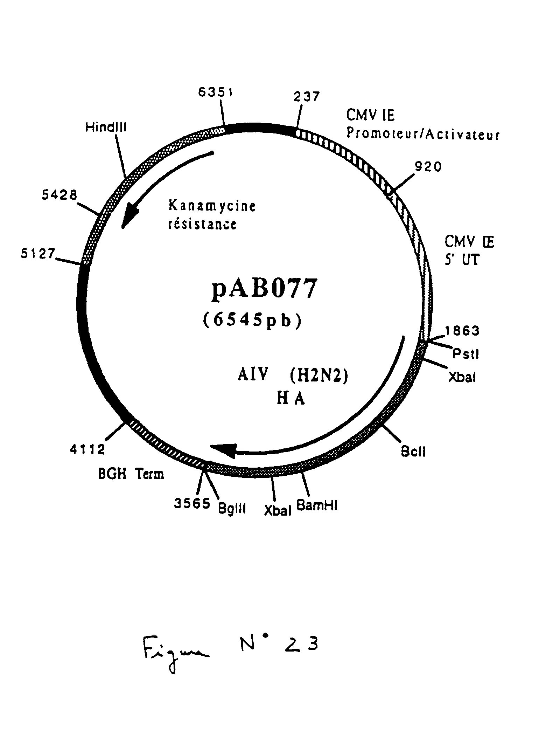

Une réaction de RT-PCR selon la technique de l'exemple 5 a été réalisée avec l'ARN génomique du virus de la peste aviaire (AIV) (Souche H2N2 Postdam) (J. Schäfer *et al*. Virology. 1993. 194. 781-788), préparé salon la technique de l'exemple 3, et avec les oligonucléotides suivants: 
AB142 (33 mer) (SEQ ID N° 37)
5'AAACTGCAGCAATGGCCATCATTTATCTAATTC 3'
 
AB143 (31 mer) (SEQ ID N° 38)
5'CGAAGATCTTCATATGCAGATTCTGCATTGC 3'
 pour isoler le gène codant pour la glycoprotéine HA du virus de la peste aviaire (souche H2N2) sous la forme d'un fragment PstI-BglII. Après purification, le produit de RT-PCR de 1709 pb a été digéré par *Pst*I et *Bgl*II pour isoler un fragment PstI-BglII de 1693 pb. Ce fragment a été ligaturé avec le vecteur pVR1012 (exemple 6), préalablement digéré avec *Pst*I et *Bgl*II, pour donner le plasmide pAB077 (6545 pb) (Figure N° 23).

### Exemple 25 : Construction du plasmide pAB078 (gène Peste aviaire HA souche H7N7)

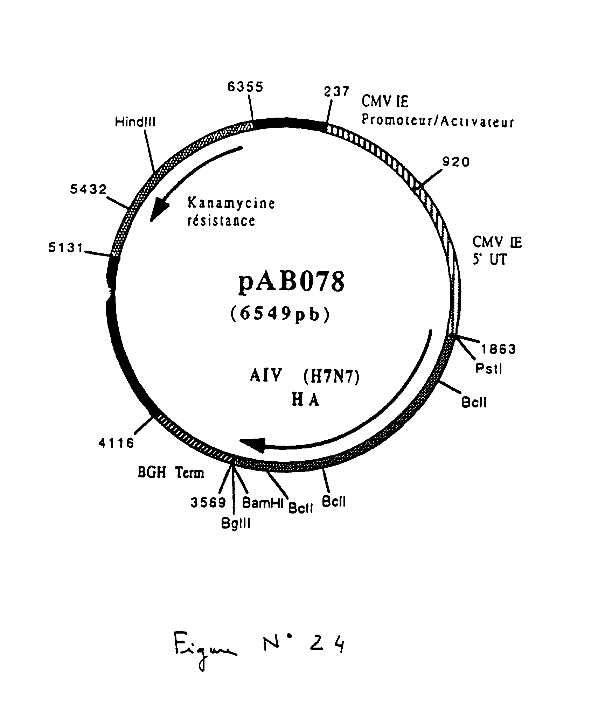

Une réaction de RT-PCR selon la technique de l'exemple 5 a été réalisée avec l'ARN génomique du virus de la peste aviaire (AIV) (Souche H7N7 Leipzig) (C. Rohm *et al*. Virology. 1995. **209.** 664-670). préparé selon la technique de l'exemple 3, et avec les oligonucléotides suivants: 
AB144 (31 mer) (SEQ ID N° 39)
5'AAACTGCAGATGAACACTCAAATCCTGATAC 3'
 
AB145 (31 mer) (SEQ ID N° 40)
5'TTTGGATCCTTATATACAAATAGTGCACCGC 3'
 pour isoler le gène codant pour la glycoprotéine HA du virus de la peste aviaire (souche H7N7) sous la forme d'un fragment PstI-BamHI. Après purification, le produit de RT-PCR de 1707 pb a été digéré par *Pst*I et *Bam*HI pour isoler un fragment PstI-BamHI de 1691 pb. Ce fragment a été ligaturé avec le vecteur pVR1012 (exemple 6), préalablement digéré avec *Pst*I et *Bam*HI, pour donner le plasmide pAB078 (6549 pb) (Figure N° 24).

### Exemple 26 : Construction du plasmide pAB088 (gène Peste aviaire NP souche H1N1)

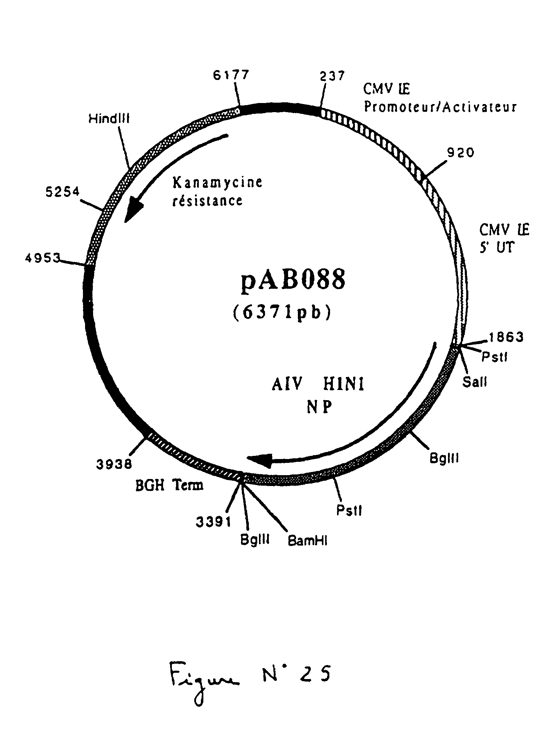

Une réaction de RT-PCR selon la technique de l'exemple 5 a été réalisée avec l'ARN génomique du virus de la grippe aviaire (AIV) (Souche H1N1 Bavaria) (M. Gammelin *et al*. Virology. 1989. **170.** 71-80), préparé selon la technique de l'exemple 3, et avec les oligonucléotides suivants: 
AB156 (32 mer) (SEQ ID N° 41)
5'CCGGTCGACATGGCGTCTCAAGGCACCAAACG 3'
 
AB158 (30 mer) (SEQ ID N° 42)
5'CGCGGATCCTTAATTGTCATACTCCTCTGC 3'
 pour isoler le gène codant pour la nucléoprotéine NP du virus de la grippe aviaire sous la forme d'un fragment Sall-BamHI. Après purification, le produit de RT-PCR de 1515 pb a été digéré par *Sal*I et *Bam*HI pour isoler un fragment SalI-BamHI de 1503 pb. Ce fragment a été ligaturé avec le vecteur pVR1012 (exemple 6), préalablement digéré avec *Sal*I et *Bam*HI, pour donner le plasmide pAB088 (6371 pb) (Figure N° 25).

### Exemple 27 : Construction du plasmide pAB079 (gène·Peste aviaire N souche H7N1)

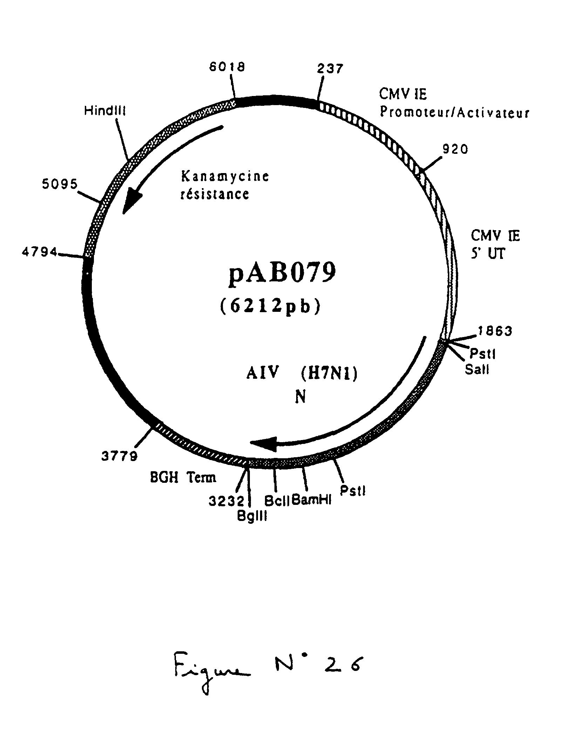

Une réaction de RT-PCR selon la technique de l'exemple 5 a été réalisée avec l'ARN génomique du virus de la peste aviaire (AIV) (Souche H7N1 Rostock) (J. Mc Cauley 1990. N° d'accès séquence sur Genbank = X52226), préparé selon la technique de l'exemple 3, et avec les oligonucléotides suivants: 
AB146 (35 mer) (SEQ ID N° 43)
5'CGCGTCGACATGAATCCAAATCAGAAAATAATAAC 3'
 
AB147 (31 mer) (SEQ ID N° 44)
5'GGAAGATCTCTACTTGTCAATGGTGAATGGC 3'
 pour isoler le gène codant pour la glycoprotéine N du virus de la peste aviaire (souche H7N1) sous la forme d'un fragment SalI-BglII. Après purification, le produit de RT-PCR de 1361 pb a été digéré par *Sal*I et *Bgl*II pour isoler un fragment SalI-BglII de 1350 pb. Ce fragment a été ligaturé avec le vecteur pVR1012 (exemple 6), préalablement digéré avec *Sal*I et *Bgl*II, pour donner le plasmide pAB079 (6212 pb) (Figure N° 26).

### Exemple 28 : Préparation et purification des plasmides

Pour la préparation des plasmides destinés à la vaccination des animaux, on peut utiliser toute technique permettant d'obtenir une suspension de plasmides purifiés majoritairement sous forme superenroulée. Ces techniques sont bien connues de l'homme de l'art. On peut citer en particulier la technique de lyse alcaline suivie de deux ultracentrifugations successives sur gradient de chlorure de césium en présence de bromure d'éthidium telle que décrite dans J. Sambrook *et al*. (*Molecular Cloning: A Laboratory Manual*. 2^{nd} Edition. Cold Spring Harbor Laboratory. Cold Spring Harbor. New York. 1989). On peut se référer également aux demandes de brevet PCT WO 95/21250 et PCT WO 96/02658 qui décrivent des méthodes pour produire à l'échelle industrielle des plasmides utilisables pour la vaccination. Pour les besoins de la fabrication des vaccins (voir exemple 17), les plasmides purifiés sont resuspendus de manière à obtenir des solutions à haute concentration ( > 2 mg/ml) compatibles avec le stockage. Pour ce faire, les plasmides sont resuspendus soit en eau ultrapure, soit en tampon TE (Tris-HCl 10 mM; EDTA 1 mM, pH 8,0).

### Exemple 29 : Fabrication des vaccins associés

Les divers plasmides nécessaires à la fabrication d'un vaccin associé sont mélangés à partir de leurs solutions concentrées (exemple 16). Les mélanges sont réalisés de telle manière que la concentration finale de chaque plasmide corresponde à la dose efficace de chaque plasmide. Les solutions utilisables pour ajuster la concentration finale du vaccin peuvent être soit une solution NACI à 0,9 % , soit du tampon PBS.
Des formulations particulières telles que les liposomes, les lipides cationiques, peuvent aussi être mises en oeuvre pour la fabrication des vaccins.

### Exemple 30 : Vaccination des poulets

Les poulets sont vaccinés avec des doses de 10, 50 ou 100 µg par plasmide. Les injections peuvent être réalisées à l'aiguille par voie intramusculaire. Les sites d'injection sont le bréchet (pour les poulets de plus de 2 semaines) et la cuisse (pour les poulets âgés d'1 jour et plus). Dans ce cas, les doses vaccinales sont administrées sous un volume de 0,1 à 0,3 ml.
Chez le poulet adulte (âgé de plus de 20 semaines), les injections sont également réalisées par voie intramusculaire en utilisant un appareil d'injection à jet liquide (sans aiguille) qui a été spécifiquement conçu pour la vaccination des poulets (par exemple, appareil AVIJET). Dans ce cas, le volume injecté est de 0,3 ml. L'injection peut être effectuée au bréchet ou au niveau de la cuisse. De même, chez le poulet adulte, les injections peuvent être réalisées à l'aiguille par voie intramusculaire, au bréchet ou à la cuisse, sous un volume de 0,3 ml. L'injection des vaccins plasmidiques peut également se faire *in ovo*. Dans ce cas, des formulations particulières, telles que citées dans l'exemple 29 peuvent être utilisées. Le volume injecté dans l'oeuf embryonné de 18 jours est compris entre 50 µl et 200 µl.

## Revendications

1. Vaccin aviaire comprenant un plasmide circulaire contenant le gène HN du virus de la maladie de Newcastle , et un véhicule approprié.

2. Vaccin selon la revendication 1, dans lequel le plasmide comprend les deux gènes F et HN du virus de la maladie de Newcastle.

3. Vaccin selon la revendication 1, qui comprend en outre un plasmide circulaire contenant le gène F du virus de la maladie de Newcastle.

4. Vaccin selon l'une quelconque des revendications 1 à 3, dans lequel le plasmide comprend un promoteur choisi dans le groupe consistant en promoteur CMV-IE, promoteur précoce SV40, promoteur tardif SV40, promoteur LTR du virus du sarcome de Rous et promoteur d'un gène de cytosquelette.

5. Vaccin selon la revendication 4, dans lequel le promoteur est un promoteur CMV-IE.

6. Vaccin selon la revendication 4, dans lequel le promoteur d'un gène du cystosquelette est le promoteur de la desmine ou le promoteur de l'actine.

7. Vaccin selon l'une quelconque des revendications 1 à 6, **caractérisé en ce qu'**il comprend en outre un plasmide contenant et exprimant un gène d'un autre pathogène aviaire.

8. Vaccin selon la revendication 7, **caractérisé en ce qu'**il comprend deux plasmides choisis dans le groupe consistant en un plasmide contenant le gène gB ou gD du virus de la Maladie de Marek, un plasmide contenant le gène VP2 du virus de la maladie de Gumboro, un plasmide contenant les gènes C + NS1 du virus de l'anémie infectieuse.

9. Vaccin selon l'une quelconque des revendications 1 à 8, **caractérisé en ce qu'**il comprend de 10 ng à 1 mg, de préférence de 100 ng à 500 µg plus préférentiellement encore de 0,1 µg à 50 µg de chaque plasmide.

10. Utilisation d'un ou de plusieurs plasmides tels que décrits dans l'une quelconque des revendications 1 à 9, pour la fabrication d'un vaccin aviaire destiné à vacciner, contre la maladie de Newcastle, la maladie de Marek, la maladie de Gumboro et/ou l'anémie infectieuse, des animaux primo-vaccinés au moyen d'un premier vaccin choisi dans le groupe consistant en vaccin entier vivant, vaccin entier inactivé, vaccin de sous-unité, vaccin recombinant, ce premier vaccin présentant le ou les antigène(s) codé(s) par la ou les plasmide(s) ou antigène(s) assurant une protection croisée.

11. Kit de vaccination regroupant un vaccin polynucléotidique selon l'une quelconque des revendications 1 à 9 et un vaccin aviaire choisi dans le groupe consistant en vaccin entier vivant, vaccin entier inactivé, vaccin de sous-unité, vaccin recombinant, ce premier vaccin présentant l'antigène codé par le vaccin polynucléocidique ou un antigène assurant une protection croisée, pour une administration de ce dernier en primo-vaccination et pour un rappel avec la formule de vaccin.

12. Vaccin selon l'une quelconque des revendications 1 à 9, accompagné d'une notice indiquant que ce vaccin polynucléotidique est utilisable en rappel d'un premier vaccin aviaire choisi dans le groupe consistant en vaccin entier vivant, vaccin entier inactivé, vaccin de sous-unité, vaccin recombinant, ce premier vaccin présentant l'antigène codé par le vaccin polynucléotidique ou un antigène assurant une protection croisée.

13. Utilisation d'un plasmide tel que décrit dans l'une quelconque des revendications 1 à 6, pour la fabrication d'un vaccin destiné à vacciner l'espèce aviaire contre le virus de la maladie de Newcastle en association avec un vaccin entier vivant ou inactivé, recombinant ou de sous-unité dirigé contre une autre pathologie aviaire.

## Claims

1. An avian vaccine comprising a circular plasmid containing the HN gene of Newcastle disease virus and a suitable vehicle.

2. A vaccine according to claim 1, in which the plasmid comprises the two F and HN genes of Newcastle disease virus.

3. A vaccine according to claim 1, which further comprises a circular plasmid containing the F gene of Newcastle disease virus.

4. A vaccine according to any one of claims 1 to 3, in which the plasmid comprises a promoter selected from the group consisting of the CMV-IE promoter, the early SV40 promoter, the late SV40 promoter, the LTR promoter for the Rous sarcoma virus and the promoter for a cytoskeleton gene.

5. A vaccine according to claim 4, in which the promoter is a CMV-IE promoter.

6. A vaccine according to claim 4, in which the promoter for a cytoskeleton gene is the promoter for desmin or the promoter for actin.

7. A vaccine according to any one of claims 1 to 6, **characterized in that** it further comprises a plasmid containing and expressing a gene of another avian pathogen.

8. A vaccine according to claim 7, **characterized in that** it comprises two plasmids selected from the group consisting of a plasmid containing the gB or gD gene of the Marek disease virus, a plasmid containing the VP2 gene of the infectious bursal disease virus, and a plasmid containing the C+NS1 genes for the infectious anaemia virus.

9. A vaccine according to any one of claims 1 to 8, **characterized in that** it comprises 10 ng to 1 mg, preferably 100 ng to 500 µg, more preferably 0.1 µg to 50 µg of each plasmid.

10. Use of one or more plasmids as described in any one of claims 1 to 9, for the production of an avian vaccine intended for vaccination against Newcastle disease, Marek disease, infectious bursal disease and/or infectious anaemia of animals prime vaccinated using a first vaccine selected from the group consisting of whole live vaccine, whole inactivated vaccine, sub-unit vaccine and recombinant vaccine, said first vaccine having antigens encoded by the plasmid(s) or antigen(s) ensuring cross protection.

11. A vaccination kit combining a polynucleotide vaccine according to any one of claims 1 to 9 and an avian vaccine selected from the group consisting of whole live vaccine, whole inactivated vaccine, sub-unit vaccine and recombinant vaccine, said first vaccine having an antigen encoded by the polynucleotide vaccine or an antigen ensuring cross protection, for administration of the latter in prime vaccination and of the polynucleotide vaccine as a booster.

12. A vaccine according to any one of claims 1 to 9, accompanied by a notice indicating that said polynucleotide vaccine can be used as a booster for a first avian vaccine selected from the group consisting of whole live vaccine, whole inactivated vaccine, sub-unit vaccine and recombinant vaccine, said first vaccine having the antigen encoded by the polynucleotide vaccine or an antigen ensuring cross protection.

13. Use of a plasmid as defined in any one of claims 1 to 6, for the production of a vaccine intended for vaccination of the avian species against Newcastle disease in combination with a whole live or inactivated vaccine, a recombinant vaccine or a sub-unit directed against a further avian disease.

## Patentansprüche

1. Vogelimpfstoff umfassend ein zirkuläres Plasmid, das das Gen HN des Virus der Newcastle-Krankheit enthält, und einen geeigneten Träger.

2. Impfstoff gemäß Anspruch 1, bei dem das Plasmid die beiden Gene F und HN des Virus der Newcastle-Krankheit umfaßt.

3. Impfstoff gemäß Anspruch 1, der ein weiteres zirkuläres Plasmid umfaßt, das das Gen F des Virus der Newcastle-Krankheit enthält.

4. Impfstoff gemäß einem der Ansprüche 1 bis 3, bei dem das Plasmid einen Promotor umfaßt, der aus der Gruppe ausgewählt ist, bestehend aus dem CMV-IE-Promotor, dem frühen SV40-Promotor, dem späten SV40-Promotor, dem LTR-Promotor des Virus des Rous-Sarkoms und dem Promotor eines Zytoskelettgens.

5. Impfstoff gemäß Anspruch 4, bei dem der Promotor ein CMV-IE-Promotor ist.

6. Impfstoff gemäß Anspruch 4, bei dem der Promotor eines Zytoskelettgens der Desmin-Promotor oder der Actin-Promotor ist.

7. Impfstoff gemäß einem der Ansprüche 1 bis 6, **dadurch gekennzeichnet daß** er außerdem ein Plasmid umfaßt, das ein Gen eines weiteren Vogel-Pathogens enthält und exprimiert.

8. Impfstoff gemäß Anspruch 7, **dadurch gekennzeichnet, daß** er zwei aus der Gruppe ausgewählte Plasmide umfaßt, die aus einem das Gen gB oder gD des Virus der Marek-Krankheit enthaltenden Plasmid, einem das Gen VP2 des Virus der Gumboro-Krankheit enthaltenden Plasmid und einem die Gene C + NS1 des Virus der infektiösen Anämie enthaltenden Plasmid besteht.

9. Impfstoff gemäß einem der Ansprüche 1 bis 8, **dadurch gekennzeichnet, daß** er 10 ng bis 1 mg, bevorzugt 100 ng bis 500 µg, noch bevorzugter 0,1 µg bis 50 µg jedes Plasmids umfaßt.

10. Verwendung eines oder mehrerer in einem der Ansprüche 1 bis 9 beschriebenen Plasmide zur Herstellung eines Vogelimpfstoffs, der zum Impfen erstgeimpfter Tiere gegen die Newcastle-Krankheit, die Marek-Krankheit, die Gumboro-Krankheit und/oder infektiöse Anämie mittels eines erstens Impfstoffs bestimmt ist, der aus der Gruppe ausgewählt ist, bestehend aus einem Lebendvollimpfstoff, inaktivierten Vollimpfstoff, Spaltimpfstoff und rekombinanten Impfstoff, wobei der erste Impfstoff das oder die Antigen(e) aufweist, das (die) durch das oder die Plasmid(e) oder Antigen(e) kodiert wird (werden), die einen Kreuzschutz sicherstellen.

11. Impfkit umfassend einen Polynukleotidimpfstoff gemäß einem der Ansprüche 1 bis 9 und einen Vogelimpfstoff, der aus der Gruppe ausgewählt ist, die aus einem Lebendvollimpfstoff, inaktivierten Vollimpfstoff, Spaltimpfstoff und rekombinanten Impfstoff besteht, wobei der erste Impfstoff das Antigen aufweist, das durch das Polynukleotidimpfstoff oder ein Antigen kodiert wird, das einen Kreuzschutz sicherstellt, zu dessen Verabfolgung als Erstimpfung und zur Wiederholung mit der Impfstofformulierung.

12. Impfstoff gemäß einem der Ansprüche 1 bis 9, der von einem Hinweis begleitet wird, der anzeigt, daß dieser Polynukleotidimpfstoff als Wiederholung eines ersten Vogelimpfstoffs verwendbar ist, der aus der Gruppe ausgewählt ist, die aus einem Lebendvollimpfstoff, inaktivierten Vollimpfstoff, Spaltimpfstoff und rekombinanten Impfstoff besteht, wobei der erste Impfstoff das Antigen aufweist, das durch das Polynukleotidimpfstoff oder ein Antigen kodiert wird, das einen Kreuzschutz sicherstellt.

13. Verwendung eines in einem der Ansprüche 1 bis 6 beschriebenen Plasmids zur Herstellung eines Impfstoffs, der zum Impfen von Vogelarten gegen das Virus der Newcastle-Krankheit zusammen mit einem gegen eine andere Vogelkrankheit gerichteten Lebendvollimpfstoff oder inaktivierten Vollimpfstoff, rekombinanten Impfstoff oder Spaltimpfstoff bestimmt ist.
